# EUROPÄISCHE PATENTANMELDUNG

(11) **EP 0 684 220 A1**
(43) Veröffentlichungstag der Anmeldung: **29.11.1995**
(21) Anmeldenummer: 95107422.8
(22) Anmeldetag: 16.05.1995
(51) Int. Cl.: C07C 45/71, C07C 45/70, C07C 47/575

(54) **Verfahren zur Herstellung von -p-Alkoxybenzaldehyden**

(30) Priorität: 27.05.1994 DE 4418508
(71) Anmelder: BAYER AG, 51368 Leverkusen (DE)
(72) Erfinder: Schnatterer, Albert, Dr., D-51373 Leverkusen (DE); Fiege, Helmut, Dr., D-51373 Leverkusen (DE); Jellito, Frank, Dr., D-51467 Bergisch Gladbach (DE); Schlee, Hans Georg, Dr., D-51379 Leverkusen (DE); Skornia, Peter, D-51427 Bergisch Gladbach (DE); Theisen, Karl-Heinz, D-51061 Köln (DE)

(57) **Zusammenfassung**

p-Alkoxybenzaldehyde werden in hohen Ausbeuten und mit Einsatz relativ geringer Mengen Alkylhalogenide erhalten, wenn man p-Hydroxybenzaldehyd mit Alkylhalogeniden in Gegenwart von basisch reagierenden Stoffen behandelt und während dieser Behandlung den pH-Wert zwischen 8,0 und 12,0 hält.

## Beschreibung

Die vorliegende Erfindung betrifft ein Verfahren zur Herstellung von p-Alkoxybenzaldehyden durch Alkylierung von p-Hydroxybenzaldehyd (p-HBA) mit Alkylhalogeniden. Im besonderen betrifft die vorliegende Erfindung ein Verfahren zur Herstellung von Anisaldehyd. Anisaldehyd ist ein bedeutender Aromastoff und ein wichtiges Zwischenprodukt zur Herstellung von Pharmazeutika, Kosmetika und Riechstoffen.

Die Herstellung von p-Alkoxybenzaldehyden durch Alkylierung von p-HBA mit Alkylhalogeniden oder Dialkylsulfaten ist prinzipiell bekannt, beispielsweise aus Ber. dt. Chem. Ges. 61, 2327 (1928) und J. Soc. Chem. Ind. 49 (1930) 408 T. Die Verfahren zur Herstellung von Anisaldehyd auf dieser Basis waren allerdings bisher durch die begrenzte Verfügbarkeit von p-HBA eingeschränkt. Mittlerweile kann mit Verfahren der selektiven Sauerstoffoxidation von p-Kresol, beschrieben beispielsweise in EP-A 12 939 und EP-A 330 036, p-HBA günstig zur Verfügung gestellt werden.

Hydroxybenzaldehyde lassen sich jedoch im Vergleich zu Phenolen schwerer alkylieren. Eine vollständige Alkylierung gelingt meist nur beim Arbeiten mit einem Überschuß an Methylierungsmitteln und Nachsetzen von Alkali.

In JP-Anm. 62-241 980 (= JP-OS 1-83042) wird beschrieben, daß man Alkalimetallsalze von p-HBA mit Methylchlorid in Wasser oder Methanol quantitativ methylieren kann, wenn nach Aufnahme von wenigstens 80 % der theoretischen Menge Methylchlorid Natronlauge nachgesetzt wird. Gemäß den dort gegebenen Beispielen reicht dazu eine einmalige Nachdosierung von Natronlauge aus. Ohne nachträgliche Alkalizugabe erreicht der Umsetzungsgrad dagegen maximal 95 %.

Bei der Oxidation von p-Kresol zu p-HBA mit Sauerstoff gemäß EP-A 12 939 und EP-A 330 036 fällt p-HBA als Alkalisalz in Alkoholen als Lösungsmittel in Gegenwart eines Überschusses an Alkali an. Ein besonders günstiges Verfahren zur Herstellung von Anisaldehyd würde sich durch direkte Methylierung in diesem Gemisch ohne Zwischenisolierung von p-HBA oder des Alkalisalzes von p-HBA ergeben. Behandelt man jedoch solche Reaktionsgemische direkt oder nach dem Abstumpfen überschüssigen Alkalis durch Säurezusatz mit Methylchlorid (gemäß der in o.a. JP-Anm. beschriebenen Art), so werden lediglich Umsätze von ca. 80 % erzielt. Selbst beim Arbeiten mit einem 1,5fachen molaren Überschuß an Methylchlorid und mit Nachdosieren von 0,5 Mol-Äquivalenten Natronlauge in mehreren Portionen bleibt der Umsatz unbefriedigend, denn er läßt sich so nur auf 85 bis 90 % steigern. Dabei laufen immer noch Nebenreaktionen in unerwünschtem Umfang ab.

Es besteht deshalb immer noch Bedarf an einem Verfahren, das die Alkylierung von p-HBA, auch in besagten Oxidationsgemischen, möglichst quantitativ ermöglicht, ohne daß gleichzeitig ein allzugroßer Überschuß an Alkylierungsmittel eingesetzt werden muß.

Es wurde nun ein Verfahren zur Herstellung von p-Alkoxybenzaldehyden der Formel 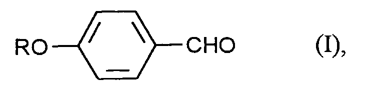
in der
- R: geradkettiges oder verzweigtes C₁-C₆-Alkyl bedeutet,
durch Behandeln von p-Hydroxybenzaldehyd mit Alkylhalogeniden der Formel

R - X (II),

in der
- X: Halogen und
- R: geradkettiges oder verzweigtes C₁-C₆-Alkyl bedeutet,
in Gegenwart von basisch reagierenden Stoffen gefunden, das dadurch gekennzeichnet ist, daß während der Alkylierung der pH-Wert zwischen 8,0 und 12,0 gehalten wird.

In den Formeln (I) und (II) kann R beispielsweise für Methyl, Ethyl, n-Propyl, i-Propyl, 1-Butyl, 2-Butyl, t.-Butyl, 1-Pentyl, i-Pentyl, 1-Hexyl oder i-Hexyl stehen und in Formel (II) X beispielsweise für Chlor, Brom oder Iod. Bevorzugt steht R für C₁-C₄-Alkyl und X für Chlor oder Brom. Besonders bevorzugt gelangt als Alkylhalogenid der Formel (II) Methylchlorid zum Einsatz.

Das erfindungsgemäße Verfahren bringt gegenüber dem Stand der Technik einen entscheidenden Fortschritt, da das Reaktionsgemisch aus der Oxidation von p-Kresol zu p-Hydroxybenzaldehyd direkt alkyliert werden kann und dabei hohe Ausbeuten erzielt werden können. Überraschenderweise gelingt es mit der pH-Steuerung die Reaktion mit hohen Umsätzen durchzuführen, ohne daß hohe Überschüsse an Alkylierungsmitteln eingesetzt werden müssen. Gleichzeitig werden Nebenreaktionen vermieden. In zu stark alkalischem Millieu läuft mit Anisaldehyd beispielsweise die Canizarro-Reaktion ab, und p-HBA tendiert bei zu niedrigem pH-Wert zu Verharzungen.

Beim erfindungsgemäßen Verfahren wird p-HBA vorzugsweise in Form von p-HBA enthaltenden Reaktionsgemischen eingesetzt, wie sie z.B. bei der katalytischen Oxidation von p-Kresol mit Sauerstoff in Alkoholen in Gegenwart von Alkali anfallen. Bevorzugt eingesetzt werden Reaktionsgemische, bei denen diese Oxidation in Gegenwart von Verbindungen des Kobalts (EP-A 12 939) und/oder von Verbindungen des Kupfers, Eisens, Cers, Mangans und/oder Nickels und/oder in Gegenwart von Kobaltphthalocyaninen (EP-A 323 290) und/oder in Gegenwart von Eisen- und/oder Manganporphyrinen (EP-A 330 036) durchgeführt wurde. Unter diesen Reaktionsgemischen sind solche besonders bevorzugt, die unter Katalyse von Eisenporphyrinen erhalten wurden.

Diese Oxidationsreaktion wird vorteilhaft in Methanol als Lösungsmittel und in Gegenwart eines Überschusses an Alkali durchgeführt. Entsprechend liegt nach beendeter Oxidation eine stark alkalische Lösung des p-HBA-Alkalisalzes in Methanol vor. Es ist bevorzugt, im Reaktionsgemisch aus der Oxidation von p-Kresol vor der Behandlung mit dem Alkylhalogenid den pH-Wert auf 10 bis 12 einzustellen, beispielsweise durch Zusatz einer Mineralsäure.

In das erfindungsgemäße Verfahren kann man auch p-HBA einsetzen, das auf andere Weise erhalten worden ist.

Das Alkylhalogenid kann zu Beginn der Alkylierung in seiner Gesamtmenge dem Reaktionsgemisch zugefügt werden, es kann auch kontinuierlich oder in beliebigen Portionen während der Reaktion zudosiert werden. Man kann das Alkylierungsmittel gegebenenfalls in verdünnter Form zusammen mit einem Lösungsmittel in das Reaktionsgemisch eintragen.

Im allgemeinen ist für einen quantitativen Umsatz des p-HBA-Alkalisalzes ein Überschuß an Alkylhalogenid notwendig. Durch die erfindungsgemäße pH-Steuerung kann jedoch der Überschuß klein gehalten werden. Aufwandmengen, die 3 Mol-Äquivalente Alkylhalogenid pro Mol-Äquivalent p-HBA-Alkalisalz überschreiten, sind deshalb nicht sinnvoll. Die Aufwandmenge an Alkylhalogenid liegt bevorzugt zwischen 1,05 und 3 Mol-Äquivalenten, besonders bevorzugt zwischen 1,1 und 2 Mol-Äquivalenten, jeweils bezogen auf das p-HBA-Alkalisalz.

Die Alkylierung kann z.B. bei 60 bis 150°C, bevorzugt bei 70 bis 130°C, durchgeführt werden.

Der Druck während der Alkylierung ist keiner spezifischen Beschränkung unterworfen. Vorzugsweise arbeitet man bei Drucken im Bereich 1 bis 30 bar, besonders bevorzugt bei 1 bis 15 bar und ganz besonders bevorzugt bei 2 bis 10 bar.

Das wesentliche Merkmal der vorliegenden Erfindung ist das Einhalten des pH-Wertes im Bereich von 8,0 bis 12,0. Bevorzugt wird der pH-Wert im Bereich 8,0 bis 11,5 gehalten. Die Einstellung des erfindungsgemäß anzuwendenden pH-Bereiches kann beispielsweise durch Arbeiten in Gegenwart von Puffersystemen, unter Ausnützung des Löslichkeitsproduktes eines Metallhydroxids oder Metalloxids oder durch kontrolliertes Nachsetzen von Base geschehen. Geeignete Puffersysteme sind beispielsweise der Carbonat/Hydrogencarbonat-Puffer. Vorteilhaft kann auch der Zusatz eines Oxids oder Hydroxids der Erdalkalimetalle sein. Diese sind im methanolischen oder gegebenenfalls wäßrigen Reaktionsmedium mäßig löslich und geben, solange sie (auch) in fester Phase vorliegen, für das erfindungsgemäße Verfahren eine geeignete OH⁻-Ionenkonzentration vor. Durch Zusatz von Kalziumoxid oder Kalziumhydroxid kann der pH-Wert der methanolischen Lösung während der Alkylierung beispielsweise konstant bei ca. 10 gehalten werden. Bevorzugt erfolgt die Einhaltung des erfindungsgemäß einzuhaltenden pH-Bereiches durch Zudosierung eines basisch reagierenden Stoffes. Erfindungsgemäß einsetzbare, basisch reagierende Stoffe, sind beispielsweise die Hydroxide und Carbonate der Alkali- und Erdalkalimetalle, beispielsweise Lithiumhydroxid, Natriumhydroxid, Kaliumhydroxid, Magnesiumhydroxid, Kalziumhydroxid, Natriumcarbonat, Kaliumcarbonat, Magnesiumcarbonat und Kalziumcarbonat. Die Hydroxide und Carbonate des Natriums und Kaliums und die Hydroxide von Magnesium und Kalzium sind bevorzugt.

Soweit Basen eingebracht werden, können diese mit oder ohne Verdünnungsmittel in das Reaktiongemisch eingesetzt werden. Bevorzugt ist die Zudosierung als Lösung oder Suspension, und bevorzugte Lösungsmittel sind dabei Wasser und Alkohole, insbesondere Wasser und Methanol.

Die einsetzbaren p-HBA-Alkalisalz-Lösungen aus der Oxidation von p-Kresol können direkt - gegebenenfalls nach Neutralisation überschüssigen Alkalis mit Säuren - mit Alkylhalogenid behandelt werden. Das erfindungsgemäße Verfahren kann auch mit Einsatzgemischen aus der Oxidation von p-Kresol durchgeführt werden, bei denen der Alkohol aus der Oxidationsreaktion gegen ein anderes Lösungsmittel, beispielsweise einen anderen Alkohol oder Wasser, ausgetauscht wurde. Neben der Alkylierung der alkoholischen Oxidationslösung ist auch die Alkylierung in Wasser vorteilhaft. Man kann auch solche p-HBA-Alkalisalz-Lösungen aus der Oxidation von p-Kresol einsetzen, bei denen vor oder nach dem Abstumpfen des überschüssigen Alkalis mit Mineralsäure vorhandene feste Bestandteile des Reaktionsgemisches (z.B. Katalysatoren, Nebenprodukte und/oder anorganische Salze) abgetrennt worden sind, z.B. durch Filtration oder Zentrifugieren.

Will man die Alkylierung in wäßrigem Medium durchführen, so kann aus der alkoholischen Oxidationslösung der Alkohol z.B. durch Anlegen eines Unterdrucks abdestilliert und gleichzeitig oder anschließend Wasser zugefügt werden. Die wäßrige Lösung kann dann nach Abstumpfen überschüssigen Alkalis dem erfindungsgemäßen Verfahren unterworfen werden. Alternativ kann das Abstumpfen überschüssigen Alkalis auch vor der Alkoholabtrennung erfolgen.

Bei der Alkylierung in wäßrigem Medium kann es vorteilhaft sein, in Gegenwart von Phasentransferkatalysatoren zu arbeiten, beispielsweise in Gegenwart von quartären Ammoniumverbindungen. Solche Phasentransferkatalysatoren, beispielsweise Tetrabutylammoniumbromid, Trimethylbenzylammoniumbromid oder Methyltrioctylammoniumbromid sind bereits in Kleiner Konzentration hoch aktiv und führen in der Regel zu einer deutlichen Erhöhung der Reaktionsgeschwindigkeit. Diese Katalysatoren können z.B. in Mengen von 0,001 bis 0,05 Mol-Äquivalenten, bevorzugt 0,001 bis 0,02 Mol-Äquivalenten, jeweils bezogen auf p-HBA eingesetzt werden.

Die Isolierung der erfindungsgemäß hergestellten Alkoxybenzaldehyde aus den beim Verfahren anfallenden Gemischen kann nach an sich bekannten Methoden erfolgen. Einige Varianten sind in den nachfolgenden Beispielen explizit dargestellt.

Wenn in den vorstehenden und nachfolgenden Darlegungen auf Anisaldehyd und dessen Herstellung Bezug genommen wird, so ist das beispielhaft für alle erfindungsgemäß herstellbaren p-Alkoxybenzaldehyde zu verstehen.

### Beispiele

### Beispiel 1

In einem 2 l Glasreaktor mit Rührer, Rückflußkühler und Gaseinleitungsrohr wurden nacheinander 216,3 g p-Kresol, 660 g Methanol, 0,15 g Cu(NO₃)₂ · 3H₂O, 280 g Natriumhydroxid und 0,12 g Eisen-tetrakis-(2,4-dimethoxyphenyl)-porphinchlorid eingegeben. Das Einsatzgemisch wurde auf 60°C erwärmt und unter Stickstoff 1 Stunde lang gerührt, wobei eine homogene Lösung entstand. Anschließend wurde unter kräftigem Rühren Sauerstoff in einem Strom von 9 l/Stunde in das Reaktionsgefäß eingeleitet. Nach 8,5 Stunden zeigte das Ansteigen des Abgasstroms die Vollständigkeit der Umsetzung an. Das Gewicht der Reaktionsmischung betrug zu diesem Zeitpunkt 1 252 g,
- Gehalt (HPLC):: 17,6 Gew.-% p-HBA,
0,5 Gew.-% p-Hydroxybenzoesäure,
kein Kresol.

Dieses Reaktionsgemisch wurde durch Zugabe von 330 ml konzentrierter Salzsäure auf einen pH-Wert von 11,0 eingestellt, in einen 2,7 l Edelstahlautoklaven mit Rührer, pH-Druckelektrode und Natronlaugedosierung überführt und auf 95°C erhitzt. Dann wurden unter Rühren insgesamt 152 g Methylchlorid während 1,5 Stunden so eindosiert, daß ein Druck von 6 bar gehalten wurde. Anschließend ließ man noch 6 Stunden bei 95°C nachreagieren. Während der Methylchloriddosierung und in der Nachrührphase wurde durch Nachdosieren von insgesamt 75 ml 45 gew.-%iger wäßriger Natronlauge in Kleinen Portionen der pH-Wert im Reaktionsgemisch zwischen 8,5 und 9,5 gehalten.

Zur Aufarbeitung des Reaktionsgemisches wurde das Methanol unter Normaldruck über eine 30 cm lange Füllkörperkolonne unter gleichzeitigem Zudosieren von 830 ml Wasser abdestilliert. Nach vollständiger Methanoldestillation bildete sich ein Zweiphasengemisch, das mit konzentrierter Salzsäure auf einen pH-Wert von 2 eingestellt wurde. Man setzte dann 120 ml Toluol zu, filtrierte das Gemisch zur Abtrennung fester Bestandteile und trennte dann die Toluol/Anisaldehyd-Phase ab. Die Wasserphase wurde nochmals mit 150 ml Toluol extrahiert. Die vereinigten Toluolphasen wurden mit 50 ml Wasser gewaschen, zur Entfernung von Wasserspuren im leichten Vakuum andestilliert und mittels HPLC-Analyse auf ihre Zusammensetzung hin untersucht. Gewicht der Toluolphase: 374 g. Der Gehalt an Anisaldehyd betrug 63,5 Gew.-%, entsprechend einer Ausbeute von 96,7 % der Theorie (bezogen auf eingesetztes p-HBA). Die Toluolphase enthielt noch 1,3 Gew.-% p-HBA.

Durch Destillation der Toluolphase im Vakuum wurde Anisaldehyd in einer Reinheit von über 98 % erhalten.

### Beispiel 2 (Vergleichsbeispiel, ohne Nachdosierung von Natronlauge)

### p-Kresol wurde wie in Beispiel 1 beschrieben oxidiert.

Das Reaktionsgemisch wurde durch Zugabe von 330 ml konzentrierter Salzsäure auf einen pH-Wert von 11,0 eingestellt, in einen 2,7 l Edelstahl-Autoklaven überführt und auf 95°C erhitzt. Unter Rühren wurden dann während 1,5 Stunden insgesamt 152 g Methylchlorid so eindosiert, daß ein Druck von 6 bar gehalten wurde. Anschließend ließ man noch insgesamt 6 Stunden bei 95°C nachreagieren. Nach dem Abkühlen des Reaktionsgemisches wurde wie in Beispiel 1 beschrieben aufgearbeitet. Gewicht der Toluolphase: 468 g. Der Gehalt an Anisaldehyd betrug 40,2 Gew.-%, entsprechend einer Ausbeute von 76,6 % der Theorie (bezogen auf eingesetzten p-HBA). Die Toluolphase enthielt noch 10,4 Gew.-% p-HBA.

### Beispiel 3 (Vergleichsbeispiel, mit Nachdosierung von Natronlauge aber ohne pH-Kontrolle)

### p-Kresol wurde wie in Beispiel 1 beschrieben oxidiert.

Das Reaktionsgemisch wurde durch Zugabe von 330 ml konzentrierter Salzsäure auf einen pH-Wert von 11,0 eingestellt, in einen 2,7 l Edelstahl-Autoklaven überführt und auf 95°C erhitzt. Unter Rühren wurden dann während 1,5 Stunden insgesamt 152 g Methylchlorid so eindosiert, daß ein Druck von 6 bar gehalten wurde. Nach Zudosierung von 100 g Methylchlorid und am Ende der Methylchloridzugabe wurden außerdem je 10 ml 45 gew.-%iger wäßriger Natronlauge eingepumpt. In der Phase des Nachreagierens wurde nochmals solche Natronlauge in 5 Portionen à 10 ml und in einer weiteren Portion zu 5 ml jeweils im Abstand von 30 Minuten zugegeben. Nach Beendigung der Natronlaugedosierung ließ man noch 3 Stunden bei 95°C nachreagieren.

Nach dem Abkühlen des Reaktionsgemisches wurde wie im Beispiel 1 beschrieben aufgearbeitet. Gewicht der Toluolphase: 463 g. Der Gehalt an Anisaldehyd betrug 43,6 Gew.-%, entsprechend einer Ausbeute von 82,2 % der Theorie (bezogen auf eingesetztes p-HBA). Die Toluolphase enthielt noch 6,2 Gew.-% p-HBA.

### Beispiel 4

p-Kresol wurde wie in Beispiel 1 beschrieben oxidiert. Das Reaktionsgemisch wurde durch Zudosieren von 138 ml 80 gew.-%iger Schwefelsäure auf einen pH-Wert von 12 gebracht. Anschließend wurde das Methanol unter Normaldruck abdestilliert und gleichzeitig wurden 1 000 ml Wasser zudosiert. Gewicht der wäßrigen Lösung: 1 862 g.

Die wäßrige Lösung wurde bei 70°C in einen 2,7 l Edelstahl-Autoklaven überführt und auf 95°C erhitzt. Dann wurden innerhalb von 3 Stunden unter Rühren insgesamt 152 g Methylchlorid so eindosiert, daß der Druck 6 bar nicht überstieg. Während der Methylchloriddosierung und der noch 5 Stunden dauernden Nachrührphase bei 95°C wurde durch Nachdosieren von insgesamt 70 ml 45 gew.-%iger wäßriger Natronlauge in kleinen Portionen der pH-Wert im Reaktionsgemisch zwischen 8,7 und 9,2 gehalten.

Nach dem Abkühlen wurde der Autoklav entspannt und das erhaltene Zweiphasengemisch mit 80 gew.-%iger Schwefelsäure auf einen pH-Wert von 2 gebracht. Man setzte dann 200 ml Toluol zu, filtrierte das Gemisch zur Abtrennung fester Bestandteile und trennte dann die Toluol/Anisaldehyd-Phase ab. Die Wasserphase wurde nochmals mit 200 ml Toluol extrahiert. Die vereinigten Toluolphasen wurden mit 50 ml Wasser gewaschen, zur Entfernung von Wasserspuren im leichten Vakuum andestilliert und mittels HPLC-Analyse auf ihre Zusammensetzung hin untersucht. Der Gehalt an Anisaldehyd entsprach einer Ausbeute von 94,8 % der Theorie (bezogen auf eingesetztes p-HBA). Die Toluolphase enthielt noch 1,5 % (bezogen auf die Einsatzmenge) unumgesetztes p-HBA.

### Beispiel 5 (Vergleichsbeispiel, ohne Nachdosierung von Natronlauge)

Es wurde verfahren wie in Beispiel 4, jedoch wurde keine Natronlauge nachdosiert. Nachdem der pH-Wert auf 6 gefallen war, wurde der Reaktor entspannt und das Reaktionsgemisch wie in Beispiel 4 aufgearbeitet. Der Gehalt an Anisaldehyd in der Toluolphase entsprach 60,4 % der Theorie (bezogen auf eingesetzten p-HBA). Außerdem enthielt die Toluolphase 21,1 % unumgesetzten p-HBA (bezogen auf eingesetzten p-HBA).

## Patentansprüche

1. Verfahren zur Herstellung von p-Alkoxybenzaldehyden der Formel 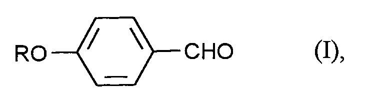 in der
R geradkettiges oder verzweigtes C₁-C₆-Alkyl bedeutet,
durch Behandeln von p-Hydroxybenzaldehyd mit Alkylhalogeniden der Formel
R - X (II),
in der
X Halogen und
R geradkettiges oder verzweigtes C₁-C₆-Alkyl bedeutet,
in Gegenwart von basisch reagierenden Stoffen, dadurch gekennzeichnet, daß während der Alkylierung der pH-Wert zwischen 8,0 und 12,0 gehalten wird.

2. Verfahren nach Anspruch 1, dadurch gekennzeichnet, daß in den Formeln (I) und (II)
R für Methyl, Ethyl, n-Propyl, i-Propyl, 1-Butyl, 2-Butyl, t.-Butyl, 1-Pentyl, i-Pentyl, 1-Hexyl oder i-Hexyl und
in Formel (II) X für Chlor, Brom oder Iod steht.

3. Verfahren nach Ansprüchen 1 und 2, dadurch gekennzeichnet, daß man p-Hydroxybenzaldehyd in Form von p-Hydroxybenzaldehyd enthaltenden Reaktionsgemischen einsetzt, wie sie bei der katalytischen Oxidation von p-Kresol mit Sauerstoff in Alkoholen in Gegenwart von Alkali anfallen.

4. Verfahren nach Ansprüchen 1 bis 3, dadurch gekennzeichnet, daß 1,05 bis 3 Mol-Äquivalente Alkylhalogenid der Formel (II) bezogen auf 1 Mol-Äquivalent p-Hydroxybenzaldehyd eingesetzt werden.

5. Verfahren nach Ansprüchen 1 bis 4, dadurch gekennzeichnet, daß die Alkylierung bei 60 bis 150°C durchgeführt wird.

6. Verfahren nach Ansprüchen 1 bis 5, dadurch gekennzeichnet, daß man den pH-Wert zwischen 8,0 und 12,0 hält, indem man in Gegenwart eines Puffersystems arbeitet oder das Löslichkeitsprodukt eines Metallhydroxids oder Metalloxids ausnutzt oder kontrolliert Base nachsetzt.

7. Verfahren nach Anspruch 6, dadurch gekennzeichnet, daß man als Base Hydroxide und/oder Carbonate von Alkali- und/oder Erdalkalimetallen einsetzt.

8. Verfahren nach Ansprüchen 1 bis 7, dadurch gekennzeichnet, daß man p-Hydroxybenzaldehyd-Lösungen aus der Oxidation von p-Kresol nach Neutralisation überschüssigen Alkalis mit Säuren einsetzt.

9. Verfahren nach Ansprüchen 1 bis 8, dadurch gekennzeichnet, daß man p-Hydroxybenzaldehyd-Lösungen aus der Oxidation von p-Kresol einsetzt, bei denen der Alkohol aus der Oxidationsreaktion gegen ein anderes Lösungsmittel ausgetauscht wurde.

10. Verfahren nach Ansprüchen 1 bis 9, dadurch gekennzeichnet, daß man bei Durchführung der Alkylierung im wäßrigen Medium in Gegenwart eines Phasentransferkatalysators arbeitet.
